(19) Europäisches Patentamt
European Patent Office
Office européen des brevets

(11) **EP 3 513 155 B1**

(12) **EUROPÄISCHE PATENTSCHRIFT**

(45) Veröffentlichungstag und Bekanntmachung des Hinweises auf die Patenterteilung:
**10.11.2021  Patentblatt 2021/45**

(21) Anmeldenummer: **17772613.0**

(22) Anmeldetag: **06.09.2017**

(51) Int Cl.:
*G01H 3/04* (2006.01)  *G01H 15/00* (2006.01)
*G01N 29/00* (2006.01)  *G01N 29/024* (2006.01)
*G01N 29/032* (2006.01)  *G01N 29/34* (2006.01)
*G01N 29/44* (2006.01)  *G01N 33/28* (2006.01)

(86) Internationale Anmeldenummer:
**PCT/EP2017/072286**

(87) Internationale Veröffentlichungsnummer:
**WO 2018/050499 (22.03.2018 Gazette 2018/12)**

(54) **VERFAHREN UND VORRICHTUNG ZUR BESTIMMUNG UND/ODER ÜBERWACHUNG DES ZUSTANDES EINES TRANSFORMATORENÖLS**

METHOD AND DEVICE FOR DETERMINING AND/OR MONITORING THE STATE OF A TRANSFORMER OIL

PROCÉDÉ ET DISPOSITIF POUR DÉTERMINER ET/OU SURVEILLER L'ÉTAT D'UNE HUILE DE TRANSFORMATEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.09.2016   DE 102016117188**

(43) Veröffentlichungstag der Anmeldung:
**24.07.2019   Patentblatt 2019/30**

(73) Patentinhaber: **Passerro GmbH**
**04299 Leipzig (DE)**

(72) Erfinder: **WROBEL, Matthias**
**81243 München (DE)**

(74) Vertreter: **advotec.**
**Patent- und Rechtsanwaltspartnerschaft**
**Tappe mbB**
**Beethovenstrasse 5**
**97080 Würzburg (DE)**

(56) Entgegenhaltungen:
DE-A1- 19 706 486        DE-A1- 19 850 799
DE-A1-102013 005 003     DE-A1-102014 104 963
DE-A1-102015 122 926

**Beschreibung**

Die Erfindung betrifft ein Verfahren und eine Vorrichtung zur Bestimmung und/oder Überwachung des Zustandes eines Transformatorenöls nach dem Obergriff der unabhängigen Ansprüche.

Vorrichtungen in der Hochspannungstechnik, wie beispielsweise Transformatoren, Kondensatoren, Petersenspulen und/oder Schalter, sind bereits aus dem Stand der Technik bekannt und dienen insbesondere, jedoch keineswegs ausschließlich, der Gewährleistung einer kontinuierlichen elektrischen Energieversorgung. Unter diesen Vorrichtungen zählen die Transformatoren zu den wichtigsten und auch kostenintensivsten Betriebsmitteln in der elektrischen Energieversorgung. Um die kontinuierliche und fehlerfreie Versorgung mit elektrischer Energie sicherzustellen sowie auch wirtschaftliche Schäden zu vermeiden, ist es wichtig, eventuell auftretende Fehler im Betrieb eines Transformators, welche Ausfälle verursachen können, rechtzeitig zu erkennen, um geeignete Maßnahmen zur Fehlerbeseitigung einzuleiten.

Die Druckschrift DE 10 2015 122926 betrifft ein System, das einen Sensor zur Messung einer Resonanzimpedanz Spektralantwort eines Spulen-Kondensator-Widerstands-Resonators und zur Korrelation der gemessenen Antwort eines oder mehrerer Spektralparameter auf eine oder mehrere Eigenschaften eines Fluids aufweist. Dabei kann die Eigenschaft das Alter, der Zustand des Fluids, die Identifikation oder die Konstellation von Komponenten im Fluid sein.

Die Druckschrift DE 197 06 486 offenbart ein elektroakustisches Verfahren zur Bestimmung des Alterungszustands eines flüssigen Mediums, insbesondere von Ö1, ausgehend von einem Ausgangszustand.

Typischerweise wird in Transformatoren eine Kombination aus einem flüssigen und einem festen Isolierstoff als Isoliermittel verwendet. Das feste Isoliermittel ist beispielsweise, jedoch keineswegs ausschließlich, Zellulosepapier und/oder eine Spanplatte. Das flüssige Isoliermittel, das Transformatorenöl, ist auch bei hohen Temperaturen stabil und dient der Isolation, der Funkenlöschung, der Schmierung und/oder der Kühlung des Transformators. Fehler an dem festen und/oder flüssigen Isolierstoff resultieren, insbesondere an derartigen flüssigkeitsgefüllten Transformatoren, dabei fast ausschließlich aus der Entwicklung von in dem Transformatorenöl gelösten Gasen sowie einer dadurch bedingten Erhöhung des Wassergehaltes. Ein Grund für die Entstehung der Gase ist beispielsweise die Zersetzung fester und/oder flüssiger Isolierstoffe, welche durch Teilentladung und Kreisströme, örtliche Überhitzungen durch Kurzschluss, hohe Übergangswiderstände, starke Wirbelströme sowie durch Lichtbogenentladungen und/oder Lichtbogenüberschläge hervorgerufen werden kann. Durch die elektrische und/oder thermische Energiezufuhr erfolgt eine Zerstörung der langkettigen Ölmoleküle, wodurch insbesondere Wasserstoff und leichte Kohlenwasserstoff-verbindungen entstehen. Zusätzlich entstehen bei der Zersetzung von Zellulose Kohlenmonoxid und Kohlendioxid, welche je nach Menge der entstandenen Gase in gelöster und/oder ungelöster Form auftreten können. So ist es auch möglich, dass Wassermoleküle entstehen, welche zu der unerwünschten Ölfeuchte führen.

Ein weiteres Problem des in dem Transformatorenöl enthaltenen Wassers besteht darin, dass das Wasser in das feste Isoliermittel, wie beispielweise Zellulosepapier und/oder die Spanplatte, eindringt und die produktionsbedingt darin enthaltenen Säuren in das Transformatorenöl auswäscht. Dadurch kommt es zu einer zusätzlichen Belastung des Transformators, wobei diese Belastung, bedingt durch mannigfaltige Ursachen, wie beispielsweise Tagesablauf-bedingte Temperaturschwankungen (z.B. zwischen Tag und Nacht), wechselnd stark oder schwach ist.

Daher ist es zur Erhaltung der Funktionalität und/oder der Gewährleistung einer kontinuierlichen elektrischen Energieversorgung wichtig, den Zustand des Transformatorenöls bzw. des Transformators zu bestimmen und/oder zu überwachen. Es ist daher nicht verwunderlich, dass aus dem Stand der Technik eine Vielzahl verschiedener Verfahren und Vorrichtungen zur Überwachung des Zustandes von Transformatorenölen bekannt sind, welche beispielsweise die in dem Transformatorenöl gelösten Gase und/oder den Wassergehalt bestimmen, da beide bekanntermaßen einen wesentlichen Einfluss auf die Durchschlagspannung und somit indirekt auf die Lebensdauer und/oder Nutzungszeit des Transformators haben. Dies liegt darin begründet, dass Wasser im Transformator zur Hydrolyse des festen Isoliermittels und somit zur Erniedrigung dessen Polymerisationsgrades führt. Allerdings weisen diese Verfahren und Vorrichtungen allesamt den Nachteil auf, dass eine Probenentnahme notwendig ist und die Bestimmung und/oder Überwachung des Zustandes des Transformatorenöls weder permanent noch online erfolgt. Auf diese Weise ist es auch nicht möglich, die Belastungsspitzen des Transformators zu erkennen. Ein weiteres Problem hierbei liegt darin, dass Transformatorenöl sehr stark hygroskopisch ist, so dass bereits die Probenentnahme zu einer Verfälschung der Messwerte führt.

Es besteht daher ein großer Bedarf an einem Verfahren und einer Vorrichtung zur Bestimmung und/oder Überwachung des Zustandes eines Transformatorenöls, und damit indirekt der Bestimmung und/oder Überwachung des Zustands einer Vorrichtung in der Hochspannungstechnik, mittels welchem bzw. welcher eine schnelle, zuverlässige und hinreichend genaue Bestimmung und/oder Überwachung des Zustandes gewährleistet ist, um einen unnötigen und auch kostenintensiven Ölwechsel zu vermeiden und gleichzeitig die kontinuierliche elektrische Energieversorgung sicherzustellen. Zudem sollten das Verfahren und die Vorrichtung kostengünstig realisierbar, zuverlässig arbeitend und für eine dauerhafte Bestimmung und/oder Überwachung geeignet sein. Die Erfindung hat sich daher die Aufgabe gestellt, ein Verfahren und eine Vorrichtung zur Bestimmung und/oder Überwachung des Zustandes eines Transformatorenöls bereitzustellen, um die oben genannten Schwierigkeiten zu überwinden und um vor allem einen vorzeitigen und/oder unnötigen Ölwechsel zu vermeiden sowie eine Ölregeneration und/oder die wartungs- und/oder reparaturbedingten Arbeiten optimal zu planen, um dadurch die Stillstandzeiten der Vorrichtung und die dadurch entstehenden Kosten auf ein Minimum zu reduzieren.

Diese Aufgabe wird auf überraschend einfache aber wirkungsvolle Weise durch ein Verfahren zur Bestimmung und/oder Überwachung des Zustandes eines Transformatorenöls sowie eine entsprechende Vorrichtung nach der Lehre der unabhängigen Hauptansprüche gelöst.

Erfindungsgemäß ist ein Verfahren zur Bestimmung und/oder Überwachung des Zustandes eines Transformatorenöls vorgeschlagen, welches die folgenden Schritte umfasst:

a) Durchführen einer akustischen Spektroskopie des Transformatorenöls, wobei mehrere Ultraschall-Sendesignale unterschiedlicher Frequenz und/oder Amplitude in das Transformatorenöl ausgesendet und entsprechende reflektierte und/oder transmittierte Ultraschall-Empfangssignale unterschiedlicher Frequenz und/oder Amplitude nach Durchlaufen des Transformatorenöls empfangen werden; und

b) Vergleichen der Ultraschall-Sendesignale mit den entsprechenden Ultraschall-Empfangssignalen, wobei eine für das Transformatorenöl charakteristische n-dimensionale Funktion ermittelt wird; und

c) Zuordnen der ermittelten charakteristischen n-dimensionalen Funktion aus Schritt b) zu einer für Transformatorenöle bekannten Referenz-Funktion entsprechender Dimension, wobei ein Referenz-Transformatorenöl bestimmt wird; und

d) Erfassen eines ersten Wertes mindestens einer charakteristischen physikalischen Eigenschaft des Transformatorenöls; und

e) Vergleichen des ersten Wertes mit einem entsprechenden Wert des Referenz-Transformatorenöls; und

f) Ermitteln des Zustandes des Transformatorenöls basierend auf dem in Schritt e) durchgeführten Vergleich.

Das erfindungsgemäße Verfahren beruht auf dem Grundgedanken, dass zur hinreichend genauen Bestimmung und/oder Überwachung des Zustandes eines Transformatorenöls die Kombination aus akustischer Spektroskopie und der Erfassung eines ersten Wertes mindestens einer charakteristischen physikalischen Eigenschaft des Transformatorenöls ausreichend ist. Erfindungsgemäß wird dabei das Transformatorenöl zuerst mittels akustischer Spektroskopie klassifiziert, indem die Ultraschall-Sendesignale mit den sich entsprechenden Ultraschall-Empfangssignalen verglichen werden. Basierend auf diesem Vergleich der entsprechenden Sende- und Empfangssignalpaare wird eine für das Transformatorenöl charakteristische n-dimensionale Funktion ermittelt, welcher wiederum einer für Transformatorenöle bekannten Referenz-Funktion entsprechender Dimension zugeordnet wird. Auf diese Weise ist es möglich, für das Transformatorenöl ein entsprechendes Referenz-Transformatorenöl zu bestimmen, welches bekannte und definierte physikalische Eigenschaften aufweist. Aufgrund dieser Klassifikation ist es ausreichend, einen Ist-Wert einer charakteristischen physikalischen Eigenschaft des Transformatorenöls zu erfassen und mit dem entsprechenden Soll-Wert des Referenz-Transformatorenöls zu vergleichen, um den Zustand des Transformatorenöls hinreichend genau zu ermitteln. Im Rahmen der Erfindung ist dabei erkannt worden, dass das Verfahren für die dauerhafte Bestimmung und/oder Überwachung des Zustandes des Transformatorenöls geeignet ist, wobei die alleinige Untersuchung des Transformatorenöls ausreichend ist und die Notwendigkeit einer Probenentnahme mit den bekannten Nachteilen vollständig entfällt.

Der Begriff "Verfahren zur Bestimmung und/oder Überwachung des Zustandes" betrifft ein Verfahren zur Ermittlung des Zustandes des Transformatorenöls, mittels welchem eine Aussage über eine restliche Nutzungsdauer, das Alter, die Durchschlagspannung und/oder das Bevorstehen eines Transformatorenöl-Wechsels bzw. einer entsprechenden Transformatorenöl-Regeneration getroffen werden kann. Dabei ist es denkbar, dass die Ermittlung einmalig oder wiederholt durchgeführt wird. Bevorzugt basiert das Verfahren auf der Ermittlung der Veränderung, bevorzugt eine Verbesserung oder eine Verschlechterung, mindestens einer charakteristischen physikalischen Eigenschaft des Transformatorenöls. Noch mehr bevorzugt wird diese Veränderung über die Zeit ermittelt, bevorzugt die Nutzungszeit, die Standzeit und/oder die Stillstandzeit. Weiterhin bevorzugt wird der Zustand des Transformatorenöls in einem regelmäßigen oder einem unregelmäßigen Intervall oder dauerhaft bestimmt, um die Veränderung der mindestens einen Eigenschaft sehr schnell erfassen zu können. Dies ist insbesondere wichtig, da es sich bei Transformatorenöl um kein statisches System handelt. Zudem lässt sich verfolgen, unter welchen Bedingungen und/oder Einflüssen die Veränderung der mindestens einen Eigenschaft des Transformatorenöls fortschreitet bzw. verlangsamt ist. Überdies lässt sich die Entstehung und/oder Ursache dieser Veränderung aufzeigen, so dass ein bevorstehendes Wartungsintervall und/oder ein bevorstehender Transformatorenöl-Wechsel bzw. eine entsprechende Transformatorenöl-Regeneration bestmöglich geplant und/oder vorhergesagt werden kann. Dabei ist es möglich, dass das erfindungsgemäße Verfahren zusätzliche Schritte enthalten kann, welche nach oder zwischen den explizit aufgeführten essentiellen Schritten a) bis f) liegen. Bevorzugt ist das Verfahren automatisierbar.

Der Begriff "Bestimmung des Zustandes" des Transformatorenöls betrifft die Ermittlung des aktuellen Zustandes des Transformatoröls. Die Bestimmung erfolgt bevorzugt semiquantitativ, quantitativ, direkt und/oder indirekt. So ist es beispielsweise möglich, mittels der Ermittlung des Zustandes des Transformatorenöls indirekt den Zustand des Transformators zu ermitteln.

Der Begriff "Überwachung des Zustands" betrifft die Verfolgung und/oder die Vorhersage des ermittelten Zustandes des Transformatorenöls. Die Überwachung ist beispielsweise, jedoch keineswegs ausschließlich, nummerisch und/oder graphisch darstellbar. Zur Erhöhung der Genauigkeit der Überwachung erfolgt diese dabei bevorzugt in einem regelmäßigen oder einem unregelmäßigen Intervall oder dauerhaft. Der Vorteil einer länger dauernden Überwachung liegt darin, dass eine Vorhersage des Zustandes des Transformatorenöls drastisch verbessert ist.

Es ist einem Fachmann verständlich, dass eine Bestimmung und/oder eine Überwachung in der Regel nicht zu 100 Prozent korrekt sein kann. Der Begriff betrifft daher eine statistisch signifikante Wahrscheinlichkeit, was die Genauigkeit der Ermittlung des Zustandes bzw. der Verfolgung und/oder Vorhersage des ermittelten Zustands betrifft. Ob eine derartige Bestimmung und/oder Überwachung statistisch signifikant ist, kann, ohne erfinderisch tätig zu werden, von einem Fachmann mittels in der Fachwelt bekannter Verfahren bestimmt werden. Beispielsweise sind statistische Evaluierungstools zu nennen, wie beispielsweise die Beurteilung des Konfidenzintervalls, des P-Wertes, des Student's T-Tests, der Mann-Whitney-Bestimmung usw. Die entsprechenden Intervalle sind mindestens 90 %, mindestens 95 %, mindestens 97 %, mindestens 98 % oder mindestens 99 % korrekt. Die P-Werte sind bevorzugt 0,1, 0,05, 0,01, 0,005 oder 0,0001. Bevorzugt ist die Bestimmung und/oder die Überwachung des Zustandes im Rahmen der vorliegenden Erfindung zu mindestens 80 %, mindestens 90 %, mindestens 95 %, mindestens 96 %, mindestens 97 %, mindestens 98 %, mindestens 99 %, mindestens 99,5 % oder 100 % korrekt.

Das erfindungsgemäße Verfahren umfasst einen Schritt a) zum Durchführen einer akustischen Spektroskopie des Transformatorenöls, wobei mehrere Ultraschall-Sendesignale unterschiedlicher Frequenz und/oder Amplitude in das Transformatorenöl ausgesendet und entsprechende reflektierte und/oder transmittierte Ultraschall-Empfangssignale unterschiedlicher Frequenz und/oder Amplitude nach Durchlaufen des Transformatorenöls empfangen werden. In einem weiteren Schritt werden die Ultraschall-Sendesignale mit den entsprechenden Ultraschall-Empfangssignalen verglichen, wobei eine für das Transformatorenöl charakteristische n-dimensionale Funktion ermittelt wird. Diesbezüglich wird aus jedem Sende- und Empfangssignalpaar die Durchlaufzeit und/oder die Frequenzverschiebung des zugeordneten Ultraschallsignals ermittelt. Zudem wird aus jedem Sende- und Empfangssignalpaar die Dämpfung des zugeordneten Ultraschallsignals, bevorzugt dessen Amplitude, beim Durchlaufen des Transformatorenöls ermittelt. Mit anderen Worten bedeutet dies, dass mit dem erfindungsgemäßen Verfahren aus jedem Sende- und Empfangssignalpaar einer entsprechenden bzw. bestimmten Frequenz im Ergebnis ein Datenpaar aus der jeweiligen Durchlaufzeit, der Frequenzverschiebung und/oder der jeweiligen Dämpfung ermittelt wird.

Im Rahmen der vorliegenden Erfindung ist dabei erkannt worden, dass es zur Beschreibung eines Sende- und Empfangssignalpaares nicht notwendig ist, alle Daten zu erfassen. So ist es beispielsweise, jedoch keinesfalls ausschließlich, nicht notwendig, die Phase des Sendesignals zu kennen, da die Phase des Sendesignals für die Ermittlung der Durchlaufzeit und/oder der Frequenzverschiebung und des Empfangs des Sendesignals beim Durchlauf durch das Transformatorenöl nicht erforderlich ist. Es müssen lediglich die Daten zur Beschreibung von Sendesignal und Empfangssignal erfasst werden, welche für die Ermittlung der Durchlaufzeit, der Frequenzverschiebung und/oder der Dämpfung relevant sind. Dies liegt darin begründet, dass sich Veränderungen im Transformatorenöl signifikant in dem Ergebnisdatenpaar aus Durchlaufzeit, Frequenzverschiebung und/oder Dämpfung abbilden, weshalb basierend auf der Messung der Durchlaufzeit und/oder der Frequenzverschiebung auch die Dispersität des untersuchten Transformatorenöls ermittelt werden kann. Dabei lässt die Kombination der Messung von Dämpfung und Dispersität des untersuchten Materials eine sehr differenzierte Charakterisierung des untersuchten Materials zu.

Erfindungsgemäß wird mit dem erfindungsgemäßen Verfahren aus jedem Sende- und Empfangssignalpaar einer entsprechenden bzw. bestimmten Frequenz im Ergebnis ein Datenpaar aus der jeweiligen Durchlaufzeit, der Frequenzverschiebung und/oder der jeweiligen Dämpfung ermittelt und gegebenenfalls in einer entsprechend eingerichteten Einrichtung zu der entsprechenden Frequenz abgespeichert. Bei der Durchführung des Verfahrens fallen sehr große Ergebnisdatensätze an, da für jede Frequenz und das zugeordnete Sende- und Empfangssignalpaar ein Ergebnisdatensatz mit der jeweiligen Durchlaufzeit, der Frequenzverschiebung und/oder der jeweiligen Dämpfung bestimmt, abgespeichert und/oder graphisch dargestellt wird. Bevorzugt ist es daher vorgesehen, dass eine Datenreduktion stattfindet, beispielsweise wird in einer Datenreduktionseinrichtung aus dem ermittelten Ergebnisdatensatz ein reduzierter Ergebnisdatensatz abgeleitet, wobei der reduzierte Ergebnisdatensatz den ermittelten Ergebnisdatensatz charakteristisch abbildet und einen geringeren Datenumfang hat. In welcher Weise die Datenreduktion durchgeführt wird, ist grundsätzlich beliebig und unterliegt dem fachmännischen Können.

Der Begriff "akustische Spektroskopie", betrifft die akustische Untersuchung eines Mediums durch Rückschlüsse aus den Veränderungen akustischer Wellen und/oder Schwingungen im Ultraschallfrequenzbereich (20 kHz - 1 GHz), wobei die Veränderungen auf den Wechselwirkungen der in dem Medium enthaltenen Moleküle mit den akustischen Wellen und/oder den Schwingungen basieren. Auf diese Weise ist es möglich, mittels der akustischen Spektroskopie die Zusammensetzung des Mediums zu untersuchen bzw. auf die Zusammensetzung des Mediums zu schließen. Das zu untersuchende Medium ist im Rahmen der Erfindung bevorzugt ein Transformatorenöl. Bevorzugt wird die akustische Spektroskopie mit Hilfe eines geeigneten Mittels durchgeführt, welches teilweise oder vollständig in dem Medium angeordnet ist und welches zur Schwingungsaussendung, zur Schwingungsübertragung, zur Schwingungsverstärkung und/oder zum Schwingungsempfang in dem Medium geeignet ist, wie beispielsweise eine Ultraschall-Sende- und/oder -Empfangseinrichtung. Es ist des Weiteren denkbar, dass das erste Mittel ein Resonator, ein Resonanzkörper, eine Resonanzkammer, ein Wandler oder eine Kombination aller zuvor genannten Einrichtungen ist.

Bevorzugt betrifft die akustische Spektroskopie Ultraschallfrequenzen, noch mehr bevorzugt Frequenzen von 75 kHz bis 750 kHz. Weiter bevorzugt wird die akustische Spektroskopie in mindestens einem Frequenzband durchgeführt. Noch mehr bevorzugt wird die akustische Spektroskopie in zwei, drei, vier, fünf, sechs, sieben, acht, neun, zehn oder mehr Frequenzbändern durchgeführt, wobei es im Rahmen der Erfindung als wesentlich erkannt worden ist, dass jedes Frequenzband eine definierte Frequenzbreite in einem definierten Frequenzbereich aufweist. Es ist daher verständlich, dass jedes Frequenzband dieselbe Frequenzbreite aufweist. So ist es beispielsweise, jedoch keinesfalls ausschließlich, möglich, die akustische Spektroskopie in 4 Frequenzbändern mit der definierten Frequenzbreite von je 75 kHz in dem definierten Frequenzbereich von 75 kHz bis 750 kHz durchzuführen, beispielsweise in der Art von Frequenzband 1 (125 kHz - 200 kHz), Frequenzband 2 (225 kHz - 300 kHz), Frequenzband 3 (325 kHz - 400 kHz) und Frequenzband 4 (525 kHz -600 kHz). Bevorzugt wird eine erneute Messung mit der Frequenz durchgeführt, welche der definierten Frequenzbreite der Frequenzbänder entspricht, wie in diesem Beispiel beispielsweise 75 kHz.

Es ist dabei erfindungsgemäß vorgesehen, dass es basierend auf dem Vergleich der entsprechenden Sende- und Empfangssignalpaare, bevorzugt aufgrund der erhaltenen entsprechenden Ergebnisdatensätze, möglich ist, eine für das Transformatorenöl charakteristische n-dimensionale Funktion ermitteln, wie beispielsweise das akustische Ungleichgewicht (AcDis; eng. *acustic disbalance*). Es ist dabei einem Fachmann bekannt, was eine n-dimensionale Funktion ist. Weiterhin wird die ermittelte charakteristische n-dimensionale Funktion einer für Transformatorenöle bekannten Referenz-Funktion entsprechender Dimension zugeordnet, wobei ein Referenz-Transformatorenöl mit definierten und bekannten physikalischen Eigenschaften bestimmt wird. Die Referenz-Funktion ist beispielsweise, jedoch keinesfalls ausschließlich, in einem Datenarchiv abgespeichert.

Im Rahmen der Erfindung betrifft der Begriff "Referenz-Funktion" bzw. "Referenz-Transformatorenöl" dabei einen Referenzwert, welcher insbesondere, jedoch keinesfalls ausschließlich, ein ermittelter und/oder ein theoretischer Wert, wie ein Laborwert und/oder ein Datenarchiveintrag, ist, sowie vor und/oder bei Inbetriebnahme des Transformatorenöls erfasst wird. Weiterhin ist es denkbar, dass ein derartiger Referenzwert einen Schwellenwert definiert, welcher bevorzugt als Obergrenze des Normalwertes der entsprechenden charakteristischen physikalischen Eigenschaft unter verschiedenen Bedingungen definiert ist. Der Wert der Obergrenze des Normalwertes kann mittels verschiedener und einem Fachmann gut bekannter Techniken bestimmt werden.

Im Anschluss wird ein erster Wert mindestens einer charakteristischen physikalischen Eigenschaft des Transformatorenöls erfasst und mit einem entsprechenden Wert des Referenz-Transformatorenöls verglichen, wobei basierend auf dem Vergleich der Zustandes des Transformatorenöls bzw. des Transformators ermittelt wird.

Der Begriff "charakteristische physikalische Eigenschaft" betrifft dabei eine für das Transformatorenöl wesentliche physikalische Eigenschaft, mittels welcher ein direkter oder indirekter Rückschluss auf den Zustand des Transformatorenöls bzw. den Transformator möglich ist. Bevorzugt verändert sich diese Eigenschaft in Abhängigkeit des Alterungsprozesses des Transformatorenöls, wobei die Veränderung bevorzugt eine Verbesserung oder eine Verschlechterung ist. Dabei ist es wichtig, dass diese Eigenschaft aus dem Referenz-Transformatorenöl hinreichend genau bekannt und/oder definiert ist, so dass es möglich ist, dessen Veränderung, beispielsweise über die Zeit, für die Bestimmung und/oder Überwachung des Zustandes zu nutzen. Der funktionale Zusammenhang ist dabei beispielsweise, jedoch keinesfalls ausschließlich, in einer 2-dimensionalen Funktion als Trend mit einem Werteverlauf über die Zeit darstellbar, beispielsweise in einer linearen Funktion, in einer logarithmischen Funktion, in einer exponentiellen Funktion, in einer logistischen Funktion, in einer polygonen Funktion und/oder in einer Mischung daraus.

Im Rahmen der Erfindung ist dabei als wesentlich erkannt worden, dass die Kombination aus akustischer Spektroskopie und der Erfassung eines ersten Wertes einer charakteristischen physikalischen Eigenschaft ausreichend ist. Dabei ist es denkbar, dass 2, 3, 4, 5, 6, 7, 8, 9, 10, 11, 12, 13, 14, 15, 16, 17, 18, 19, 20 oder mehr Werte derselben charakteristischen physikalischen Eigenschaft erfasst werden. Bevorzugt ist es denkbar, dass ein Mittelwert für den weiteren Vergleich dieses Wertes mit dem entsprechenden Wert des Referenz-Transformatorenöls verwendet wird. Alternativ ist es ebenfalls denkbar, dass 2, 3, 4, 5, 6, 7, 8, 9, 10, 11, 12, 13, 14, 15, 16, 17, 18, 19, 20 oder mehr Werte unterschiedlicher charakteristischer physikalischer Eigenschaften erfasst werden. Dies liegt darin begründet, dass eine physikalische Eigenschaft von verschiedenen Faktoren abhängig und/oder beeinflussbar ist, deren zusätzliche Erfassung zu einer Verbesserung der Bestimmung und/oder Überwachung beiträgt. Beispielsweise sind in diesem Zusammenhang die Temperatur, der Druck, die Farbe, der Brechungsindex und/oder die Feuchtigkeit denkbar.

Der Begriff "Vergleich" betrifft dabei den Vergleich sich entsprechender Werte miteinander, insbesondere des Ultraschall-Sendesignals mit dem entsprechenden Ultraschall-Empfangssignal bzw. des ersten Wertes mindestens einer charakteristischen physikalischen Eigenschaft des Transformatorenöls mit dem entsprechenden Wert des Referenz-Transformatorenöls. Dabei versteht es sich, dass ein Vergleich, wie er hier angewendet wird, sich auf einen Vergleich entsprechender Parameter und/oder Werte bezieht. So ist es beispielsweise denkbar, dass ein absoluter erster Wert der charakteristischen physikalischen Eigenschaft mit einem absoluten entsprechenden Wert des Referenz-Transformatorenöls vergleichbar ist. Gleiches gilt für relative Werte, ein Intensitätssignal und/oder ein Ultraschall-Signal. Es ist auch denkbar, den Vergleich auf der Basis eines empirisch ermittelten Modells für Referenz-Transformatorenöle durchzuführen.

Im Rahmen der Erfindung werden der Vergleich, die Zuordnung, die Erfassung und/oder die Ermittlung bevorzugt computerunterstützt durchgeführt. Für eine computerunterstützte Durchführung dieser Schritte, beispielsweise der Schritte b), c), e) und/oder f), sind alle einem Fachmann bekannten Mittel denkbar, wie beispielsweise Computer und/oder ein Computerprogramm. Ein Computerprogramm kann zusätzlich das entsprechende Ergebnis evaluieren, beispielsweise automatisch eine Beurteilung des Wertes liefern. Dabei ist bevorzugt mittels des in Schritt b) durchgeführten Vergleichs eine Zuordnung der entsprechenden charakteristischen n-dimensionalen Funktion des Transformatorenöls zu einer Referenz-Funktion möglich. Weiterhin ist bevorzugt mittels des in Schritt e) durchgeführten Vergleichs eine Ermittlung des Zustands des Transformatorenöls möglich. Es ist weiterhin beispielsweise denkbar, dass die Schritte b), c), e) und/oder f) von einer Auswerte-, einer Analyse- und/oder einer Evaluierungseinheit unterstützt sind. Bevorzugt ist es auch möglich, zeitlich aufeinanderfolgende Werte einer charakteristischen physikalischen Eigenschaft bzw. Ultraschallsende- und/oder Ultraschallempfangssignale in dem Vergleich zu berücksichtigen, so dass basierend auf diesem Vergleich eine Vorhersage getroffen werden kann, wie sich der Zustand in zeitlicher Abhängigkeit verändert.

Im Rahmen der Erfindung ist es verständlich, dass das Ergebnis des Vergleichs, d.h. die Ermittlung und/oder Evaluierung des Zustandes des Transformatorenöls, direkt oder indirekt von der Zuordnung der charakteristischen n-dimensionalen Funktion zu der Referenz-Funktion und der charakteristischen physikalischen Eigenschaft abhängig ist. Somit ist es denkbar, dass eine geringe und nicht signifikante, eine große und signifikante und/oder keine Veränderung des ersten Wertes der charakteristischen physikalischen Eigenschaft des Transformatorenöls im Hinblick auf den entsprechenden Wert des Referenz-Transformatorenöls indikativ für einen bestimmten Zustand ist. Eine Veränderung der charakteristischen physikalischen Eigenschaft kann bevorzugt eine Verbesserung und/oder eine Verschlechterung dieser sein. In diesem Zusammenhang ist es denkbar, dass das Ergebnis des Vergleichs als zeitliche Angabe, beispielsweise in Jahren, Monaten, Tagen, Stunden und/oder Minuten, in einem absoluten Wert und/oder einem relativen Wert ausgebbar ist.

Der Begriff "Transformatorenöl" betrifft einen bei hohen Temperaturen stabilen, flüssigen Isolierstoff, welcher der Isolation, der Funkenlöschung, der Schmierung und/oder der Kühlung einer Vorrichtung in der Hochspanungstechnik, wie beispielsweise eines Transformators, eines Kondensators und/oder eines Schalters, dient. Der flüssige Isolierstoff ist beispielweise, jedoch keineswegs ausschließlich, ein hochraffiniertes Mineralöl, ein verflüssigtes Gas (GTL), ein dünnflüssiges Silikonöl, ein Natural-Öl, ein Pflanzenöl, ein synthetischer organischer Ester, wie ein gesättigter Pentaerythrittetrafettsäureester, und/oder eine Aminosäureverbindung.

Mittels des erfindungsgemäßen Verfahrens ist es somit möglich, den Zustand des Transformatorenöls einfach, schnell und zuverlässig zu bestimmen und/oder zu überwachen, um beispielsweise eine Aussage über den Zustand der entsprechenden Vorrichtung in der Hochspannungstechnik geben zu können. Dabei ist es möglich, diese Bestimmung und/oder Überwachung bei einer im Betrieb befindlichen Vorrichtung, d.h. online, durchzuführen. Vorteilhafterweise ist das Verfahren derart ausgestaltet, dass auf eine Probenentnahme mit den bekannten Nachteilen vollständig verzichtet werden kann. Auf diese Weise ist es möglich, einen vorzeitigen und/oder unnötigen sowie kostenintensiven Ölwechsel zu vermeiden und gleichzeitig die kontinuierliche elektrische Energieversorgung zu jeder Zeit sicherzustellen. Zudem ist es möglich, die für eine Wartung, Regeneration und/oder Reparatur erforderlichen Stillstandzeiten der Vorrichtung optimal zu planen, um unnötige Stillstandzeiten und/oder Kosten zu vermeiden.

Vorteilhafte Weiterbildungen der Erfindung, welche einzeln oder in Kombination realisierbar sind, sind in den Unteransprüchen dargestellt.

In einer Weiterbildung der Erfindung ist es denkbar, dass das Verfahren zusätzlich umfasst:

g) Darstellen der in Schritt f) durchgeführten Ermittlung.

Mittels dieser Ausgestaltung ist es möglich, den Zustand des Transformatorenöls numerisch und/oder graphisch darzustellen, um auf diese Weise eine Vereinfachung des Verständnisses der Ermittlung in Schritt f) zu erreichen. Einem Fachmann sind geeignete Mittel zur Darstellung einer Ausgabe eines Wertes bekannt. So ist es beispielsweise, jedoch keinesfalls ausschließlich, möglich, eine restliche Nutzungsdauer, die Durchschlagspannung und/oder das Bevorstehen eines Transformatorenöl-Wechsels oder einer Transformatorenöl-Regeneration dazustellen. Der Schritt g) kann von einer Ausgabeeinheit unterstützt sein.

In einer weiteren Ausgestaltung der Erfindung ist es denkbar, dass nach dem Schritt d) ein zusätzlicher Schritt d1) umfasst ist:

d1) Erfassen mindestens eines zweiten Wertes mindestens einer charakteristischen physikalischen Eigenschaft des Transformatorenöls.

Die Erfassung eines zweiten Wertes bietet den Vorteil, dass eine bessere Annäherung an den funktionalen Zusammenhang der entsprechenden Eigenschaft und damit eine drastische Verbesserung der Ermittlung in Schritt f) erreichbar ist. Auf diese Weise ist beispielsweise, jedoch keinesfalls ausschließlich, eine bessere Aussage über die Krümmung der Funktion treffbar, wobei gilt, dass die Genauigkeit der Ermittlung in Schritt d) und/oder Schritt d1) mit zunehmender Anzahl an erfassten Werten steigt. Bevorzugt ist die Erfassung eines zweiten Wertes denkbar, indem ein zweiter Wert einer anderen charakteristischen physikalischen Eigenschaft erfasst wird. Alternativ bevorzugt ist es denkbar, dass ein zweiter Wert der bereits in Schritt d) erfassten Eigenschaft bestimmt wird.

In einer weiteren Ausgestaltung der Erfindung ist es denkbar, dass mittels akustischer Spektroskopie die Dichte, die Viskosität, die relative und/oder die absolute Menge eines Inhibitors und/oder einer Säure des Transformatorenöls und/oder ein Gemisch daraus bestimmt werden. Dabei ist im Rahmen der Erfindung erkannt worden, dass sich weitere Vorteile des erfindungsgemäßen Verfahrens aus dem breiten Anwendungsspektrum der akustischen Spektroskopie ergeben. So ist bevorzugt die Bestimmung der relativen und/oder der absoluten Menge eines Inhibitors und/oder einer Säure, bevorzugt eine nicht über die pH-Wert-Messung bestimmbare kurz- oder langkettige Säure, in dem Transformatorenöl wichtig, welche auf aktuellem Stand der Technik lediglich mittels Probenentnahme und nachfolgender Titration erfolgt. Die Verwendung eines Inhibitors, wie beispielsweise Diphenyldisulfit, dient dem Schutz vor Korrosion, indem Diphenyldisulfit beispielsweise korrosiven Schwefel im Tranformatorenöl bindet und auf diese Weise zuverlässig die Bildung von Schwefelsäure verhindert und/oder unterdrückt. Allerdings sind derartige Inhibitoren in der Regel hoch giftige Verbindungen, so dass verständlicherweise die Bestimmung von deren relativer und/oder absoluter Menge mit einem enormen Aufwand und hohen Kosten verbunden ist. Mittels des erfindungsgemäßen Verfahrens sind diese jedoch, neben anderen, einfach, schnell, zuverlässig und gefahrlos bestimmbar, was die enormen Vorteile des Verfahrens noch einmal unterstreicht.

In noch einer Ausgestaltung der Erfindung ist es denkbar, dass die charakteristische physikalische Eigenschaft die Schallgeschwindigkeit, die Dichte, die Farbe, der Brechungsindex, die Schallabsorption, die Temperatur, die Oberflächenspannung, die Viskosität, die relative und/oder absolute Feuchte bzw. Sättigung, der Verlustfaktor, der Säuregehalt, die elektrische Konstante, die elektrische Leitfähigkeit und/oder die Konzentration mindestens eines Fluides ist. Die zuvor genannten charakteristischen physikalischen Eigenschaften sind einem Fachmann gut bekannt, ebenso deren Bestimmung und/oder Berechnung. Zudem sind weitere, hier nicht aufgeführte, Eigenschaften denkbar.

Es ist weiterhin denkbar, dass der Zustand ausgewählt ist aus der Liste aus der restlichen Nutzungsdauer, dem Alterungszustand, der Durchschlagspannung und/oder dem Bevorstehen eines Transformatorenöl-Wechsels oder einer Transformatorenöl-Regeneration.

Der Begriff "restliche Nutzungsdauer" betrifft die Ermittlung der noch verbleibenden Zeit des Transformatorenöls, in welcher die Funktionalität, Isolierfähigkeit und/oder Schutzfunktion für die entsprechende Vorrichtung in der Hochspannungstechnik noch besteht. Durch Ermittlung dieses Wertes lässt sich beispielsweise bestimmen und/oder überwachen, wann das Transformatorenöl zu warten, zu reparieren, zu regenerieren und/oder auszutauschen ist. Es lässt sich zudem beispielsweise überwachen, wie sich die Funktionalität, Isolierfunktion und/oder Schutzfunktion des Transformatorenöls über die Zeit verändert. Die restliche Nutzungsdauer ist bevorzugt in Form einer Zeiteinheit darstellbar, wie beispielsweise in Jahren, Monaten, Tagen, Stunden und/oder Minuten, sowie als ein relativer Wert.

Der Begriff "Alterungszustand" beschreibt beispielsweise eine Ermittlung des maximalen Alters, der Funktionalität, der Isolierfähigkeit und/oder der Schutzfunktion des Transformatorenöls und/oder der Ausführwahrscheinlichkeit der entsprechenden Vorrichtung. Mittels dieser Werte ist es beispielweise möglich, Schlüsse darauf zu ziehen, wann eine Wartung, eine Reparatur bzw. eine Regeneration und/oder Austausch sinnvoll und/oder nötig ist, um sicherzustellen, dass es zu keinen alterungsbedingten Ausfällen der entsprechenden Vorrichtung und damit zu keinem Ausfall der elektrischen Energieversorgung mit den entsprechenden wirtschaftlichen Nachteilen kommt. Der Alterungszustand ist bevorzugt in Form einer Zeiteinheit darstellbar, wie beispielsweise in Jahren, Monaten, Tagen, Stunden und/oder Minuten, sowie als ein relativer Wert.

Der Begriff "Durchschlagspannung", abgekürzt BDV (engl. *breakdown voltage*) betrifft diejenige elektrische Feldstärke im Transformatorenöl, welche in diesem höchstens herrschen darf, ohne dass es zu einem Spannungsdurchschlag, Lichtbogen und/oder Funkenschlag und den damit verbundenen Ausfällen sowie Nachteilen kommt. Die Durchschlagspannung ist von verschiedenen Faktoren abhängig. Die Durchschlagspannung ist bevorzugt graphisch und/oder in Form eines absoluten Wertes darstellbar, welcher konform mit der gültigen DIN-Norm ist, wie beispielsweise mit der DIN EN 60243-1:2012-05.

Der Begriff "Bevorstehen eines Transformatorenöl-Wechsels oder einer Transformatorenöl-Regeneration" betrifft die Ermittlung der noch verbleibenden Zeit des Transformatorenöls, bis dieses zu warten, zu reparieren, zu regenerieren und/oder auszutauschen ist. Es lässt sich zudem beispielsweise überwachen, wie sich die Funktionalität, Isolierfähigkeit und/oder Schutzfunktion des Transformatorenöls über die Zeit verändert. Das Bevorstehen eines Transformatorenöl-Wechsels oder einer Transformatorenöl-Regeneration ist bevorzugt in einer Zeiteinheit darstellbar, wie beispielsweise in Jahren, Monaten, Tagen, Stunden und/oder Minuten, sowie als ein relativer Wert.

Es wird davon ausgegangen, dass die Definitionen und/oder die Ausführungen der oben genannten Begriffe für alle in dieser Beschreibung im Folgenden beschriebenen Aspekte gelten, sofern nichts anderes angegeben ist.

Erfindungsgemäße ist weiterhin eine Vorrichtung zur Bestimmung und/oder Überwachung des Zustandes eines Transformatorenöls nach einem der Verfahrensansprüche vorgeschlagen, welche die folgenden Schritte umfasst:

a) ein erstes Mittel zum Durchführen einer akustischen Spektroskopie des Transformatorenöls, wobei das erste Mittel eine Ultraschall-Sendeeinrichtung zum Aussenden mehrerer Ultraschall-Sendesignale unterschiedlicher Frequenz und/oder Amplitude in das Transformatorenöl und eine Ultraschall-Empfangseinrichtung zum Empfangen entsprechender reflektierter und/oder transmittierter Ultraschall-Empfangssignale unterschiedlicher Frequenz und/oder Amplitude nach Durchlaufen des Transformatorenöls umfasst; und

b) eine erste Evaluierungseinheit zum Vergleichen der Ultraschall-Sendesignale mit den entsprechenden Ultraschall-Empfangssignalen, wobei eine für das Transformatorenöl charakteristische n-dimensionale Funktion ermittelbar ist; und

c) eine erste Auswerteeinheit zum Zuordnen der ermittelten charakteristischen n-dimensionalen Funktion aus Schritt b) zu einer für Transformatorenöle bekannten Referenz-Funktion entsprechender Dimension, wobei ein Referenz-Transformatorenöl bestimmbar ist; und

d) ein zweites Mittel zum Erfassen eines ersten Wertes mindestens einer charakteristischen physikalischen Eigenschaft des Transformatorenöls; und

e) eine zweite Evaluierungseinheit zum Vergleichen des ersten Wertes mit einem entsprechenden Wert des Referenz-Transformatorenöls; und

f) eine zweite Auswerteeinheit zum Ermitteln des Zustandes des Transformatorenöls basierend auf dem in Schritt e) durchgeführten Vergleich.

Die erfindungsgemäße Vorrichtung ist dabei bevorzugt selbstlernend und/oder selbstkalibrierend, um eine bestmögliche Bestimmung und/oder Überwachung des Zustandes des Transformatorenöls zu erhalten.

Der Begriff "erstes Mittel" betrifft ein beliebiges, einem Fachmann aus dem Stand der Technik bekanntes Mittel, welches im Ultraschallfrequenzbereich (20 kHz - 1 GHz) zur Schwingungsaussendung, zur Schwingungsübertragung, zur Schwingungsverstärkung und/oder zum Schwingungsempfang in einem Medium geeignet ist, wobei das zu untersuchende Medium bevorzugt ein Transformatorenöl ist. Das Mittel ist dabei bevorzugt teilweise oder vollständig in dem Medium angeordnet. Bevorzugt ist das erste Mittel eine Ultraschall-Sendeeinrichtung zum Aussenden mehrerer Ultraschall-Sendesignale unterschiedlicher Frequenz und/oder Amplitude in das Transformatorenöl und/oder eine Ultraschall-Empfangseinrichtung zum Empfangen entsprechender reflektierter und/oder transmittierter Ultraschall-Empfangssignale unterschiedlicher Frequenz und/oder Amplitude nach Durchlaufen des Transformatorenöls. Es ist des Weiteren denkbar, dass das erste Mittel ein Resonator, ein Resonanzkörper, eine Resonanzkammer, ein Wandler oder eine Kombination aller zuvor genannten Einrichtungen ist.

Der Begriff "zweites Mittel" betrifft ein beliebiges, einem Fachmann aus dem Stand der Technik bekanntes Mittel, welches dazu geeignet ist, die charakteristische physikalische Eigenschaft des Transformatorenöls zu einem Zeitpunkt zu erfassen. Bevorzugt erfolgt gleichzeitig die Erfassung des Zeitpunktes.

Der Begriff "Evaluierungseinheit" betrifft eine Einheit, welche zum Vergleichen der erfassten Signale, Werte und/oder Zustandswerte geeignet ist. Geeignete Evaluierungseinheiten sind einem Fachmann bekannt, beispielsweise ein Computer und/oder ein Computerprogramm. Ein Computerprogramm kann zusätzlich das Ergebnis des Vergleichs beurteilen.

Der Begriff "Auswerteeinheit" betrifft eine Einheit, welche zur Auswertung bzw. Ermittlung des Zustandes des Transformatorenöls geeignet ist. Beispielsweise ist die Auswerteeinheit ein Computer und/oder ein Computerprogramm.

Die erfindungsgemäße Vorrichtung weist den Vorteil auf, dass diese eine hinreichend genaue Sensibilität zur Bestimmung und/oder Überwachung des Zustandes des Transformatorenöls im laufenden Betrieb, d.h. online, aufweist sowie zugleich robust genug ist, um unter den alltäglichen Bedingungen eines arbeitenden Transformators langfristig zu bestehen.

Vorteilhafte Weiterbildungen der Erfindung, welche einzeln oder in Kombination realisierbar sind, sind in den Unteransprüchen dargestellt.

In einer Weiterbildung der Erfindung ist es denkbar, dass zusätzlich eine Ausgabeeinheit zur Darstellung der mittels der zweiten Ausgabeeinheit durchgeführten Ermittlung umfasst ist. Der Begriff "Ausgabeeinheit" betrifft eine Einheit, welche zur Darstellung des ermittelten Zustands geeignet ist. Mittels dieser Ausgestaltung ist es möglich, den Zustand des Transformatorenöls numerisch und/oder graphisch darzustellen, um auf diese Weise eine Vereinfachung des Verständnisses der Ermittlung im Schritt f) zu erreichen. Einem Fachmann ist eine geeignete Ausgabeeinheit zur Darstellung bekannt.

In einer weiteren Ausgestaltung der Erfindung ist es denkbar, dass ein zusätzliches Mittel zum Erfassen mindestens eines zweiten Wertes mindestens einer charakteristischen physikalischen Eigenschaft des Transformatorenöls umfasst ist. Ein derartiges Mittel zur Erfassung eines weiteren Zustandswertes ist dem Fachmann bekannt. Bevorzugt ist dieses Mittel dasselbe Mittel wie in Schritt d), d.h. das zweite Mittel. Alternativ bevorzugt ist dieses Mittel ein anderes Mittel als das in Schritt d), d.h. ein drittes Mittel.

Es ist weiterhin denkbar, dass mittels des ersten Mittels die Dichte, die Viskosität, die relative und/oder die absolute Menge eines Inhibitors und/oder einer Säure des Transformatorenöls und/oder ein Gemisch daraus bestimmbar sind.

In einer Weiterbildung der vorliegenden Erfindung ist es denkbar, dass das erste Mittel und/oder das zweite Mittel die Schallgeschwindigkeit, die Dichte, die Farbe, den Brechungsindex, die Schallabsorption, die Temperatur, die Oberflächenspannung, die Viskosität, die relative und/oder absolute Feuchte bzw. Sättigung, den Verlustfaktor, den Säuregehalt, die elektrische Konstante, die elektrische Leitfähigkeit und/oder die Konzentration mindestens eines Fluides erfasst.

In noch einer Weiterbildung ist es denkbar, dass der Zustand ausgewählt ist aus der Liste aus der restlichen Nutzungsdauer, dem Alterungszustand, der Durchschlagspannung und/oder dem Bevorstehen eines Transformatorenöl-Wechsels oder einer Transformatorenöl-Regeneration.

In einer alternativen Ausgestaltung ist es denkbar, dass das erste Mittel, das zweite Mittel, die erste Evaluierungseinheit, die zweite Evaluierungseinheit, die erste Auswerteeinheit, die zweite Auswerteeinheit und/oder die Ausgabeeinheit in einem Bauteil anordenbar sind. Diese Ausgestaltung bietet den Vorteil, dass die Vorrichtung kompakt und sehr leicht händelbar sowie gut transportierbar ist.

In einer alternativen Ausgestaltung dieser Weiterbildung ist es denkbar, dass das Bauteil eine Messkammer, ein Stick und/oder ein Adapter ist. Diese Ausgestaltung bietet den Vorteil, dass die Vorrichtung einfach, schnell und zuverlässig mit einer Vorrichtung in der Hochspannungstechnik, wie beispielsweise einem Transformator, verbindbar ist. Weiterhin bevorzugt ist die Verbindung der Vorrichtung direkt, beispielsweise kabelgebunden oder über einen Adapter.

In einer weiteren Ausgestaltung ist es denkbar, dass die Vorrichtung eine Heizeinrichtung umfasst. Diese Ausgestaltung bietet den Vorteil, dass die Vorrichtung vor Durchführung der ersten Messung aufheizbar ist, so dass sichergestellt ist, dass die in der Vorrichtung befindlichen Mittel immer trocken sind. Dies trägt maßgeblich zur Verbesserung der gemessenen Werte bei, da Verfälschungen in der Regel aufgrund einer Durchfeuchtung der Mittel bedingt sind. Einem Fachmann sind derartige Heizeinrichtungen, wie beispielsweise eine Heizschlange und/oder ein Peltier-Element, gut bekannt.

Erfindungsgemäß ist weiterhin eine Vorrichtung in der Hochspannungstechnik, insbesondere ein Transformator, ein Kondensator, eine Petersenspule und/oder ein Schalter, umfassend Transformatorenöl und eine Einrichtung zur Verbindung mit der Vorrichtung nach einem der vorhergehenden Ansprüche vorgeschlagen, wobei die Verbindung direkt erfolgt.

Der Begriff "direkte Verbindung" betrifft eine beliebige, unmittelbare Verbindung der Einrichtung mit der Vorrichtung. Eine derartige Verbindung ist beispielsweise, jedoch keineswegs ausschließlich, mittels einer Ausnehmung und/oder eines Vorsprungs seitens der Einrichtung und einer entsprechend ausgebildeten Vorrichtung realisierbar. Es ist weiterhin denkbar, dass eine direkte Verbindung eine USB-, TCP/IP-, MODBUS-Verbindung oder eine andere kabelgebundene oder kabellose Verbindung ist. Mittels dieser Ausgestaltung ist es einfach, schnell und zuverlässig möglich, die Vorrichtung zur Bestimmung des Zustandes des Transformatorenöls mit einer Vorrichtung in der Hochspannungstechnik zu verbinden, um schnell, zuverlässig und hinreichend genau den Zustand derselben zu bestimmen und/oder zu überwachen.

Weitere Einzelheiten, Merkmale und Vorteile der Erfindung ergeben sich aus der nachfolgenden Beschreibung der bevorzugten Ausführungsbeispiele in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktion einander entsprechende Elemente.

Im Einzelnen zeigen:

Fig. 1    eine isometrische Darstellung einer ersten Ausführungsform einer erfindungsgemäßen Vorrichtung mit Schutzabdeckung; und

Fig. 2    eine weitere isometrische Darstellung der ersten Ausführungsform einer erfindungsgemäßen Vorrichtung ohne Schutzabdeckung; und

Fig. 3    eine Explosionsdarstellung der ersten Ausführungsform einer erfindungsgemäßen Vorrichtung ohne Schutzabdeckung; und

**Fig. 4** eine isometrische Darstellung des Sensorbereichs der ersten Ausführungsform einer erfindungsgemäßen Vorrichtung mit unterschiedlichen Befestigungsbereichen (Fig. 4a und 4b); und

**Fig. 5** eine isometrische Darstellung einer zweiten Ausführungsform einer erfindungsgemäßen Vorrichtung; und

**Fig. 6** eine isometrische Darstellung eines Sensorbereichs der zweiten Ausführungsform einer erfindungsgemäßen Vorrichtung; und

**Fig. 7** eine weitere isometrische Darstellung eines Sensorbereichs der zweiten Ausführungsform einer erfindungsgemäßen Vorrichtung; und

**Fig. 8** eine Matrix-Grafik einer beispielhaften Berechnung der Bestimmung der Durchschlagspannung eines Transformatorenöls.

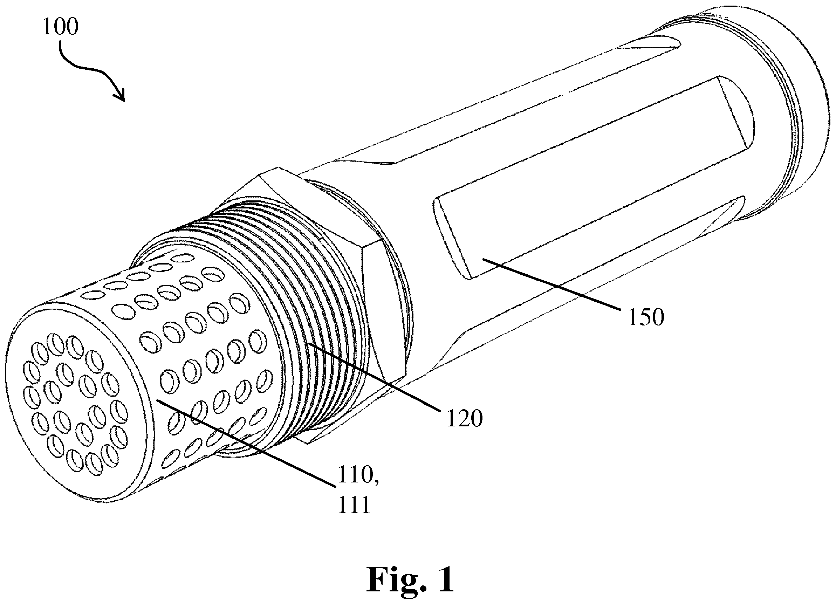

In der **Fig. 1** ist eine erste Ausführungsform einer erfindungsgemäßen Vorrichtung 100 zur Bestimmung und/oder Überwachung des Zustandes eines Transformatorenöls in isometrischer Darstellung gezeigt, welche in der Form eines Sticks 100 ausgebildet ist. Wie deutlich in der Fig. 1 zu erkennen ist, weist der Stick 100 einen mit einer Schutzabdeckung 111 versehenen Sensorbereich 110 auf. Zudem ist an dem Stick 100 ein Befestigungsbereich 120 zur sicheren Befestigung des Sticks 100 an einem Transformator vorgesehen, welcher beispielsweise in der Form eines 1,5-Zoll-Rohrgewindes ausgebildet ist. Das Gehäuse 150 des Sticks 100 dient neben der Isolierung auch dem Schutz der Elektronik, insbesondere vor einem ungewollten elektrischen und/oder elektromagnetischen Effekt, sowie als Kommunikationsvorrichtung und ist aus einem beliebigen Material denkbar. Bevorzugt ist das Gehäuse 105 aus einem die elektromagnetische Verträglichkeit gewährleistenden metallischen Werkstoff.

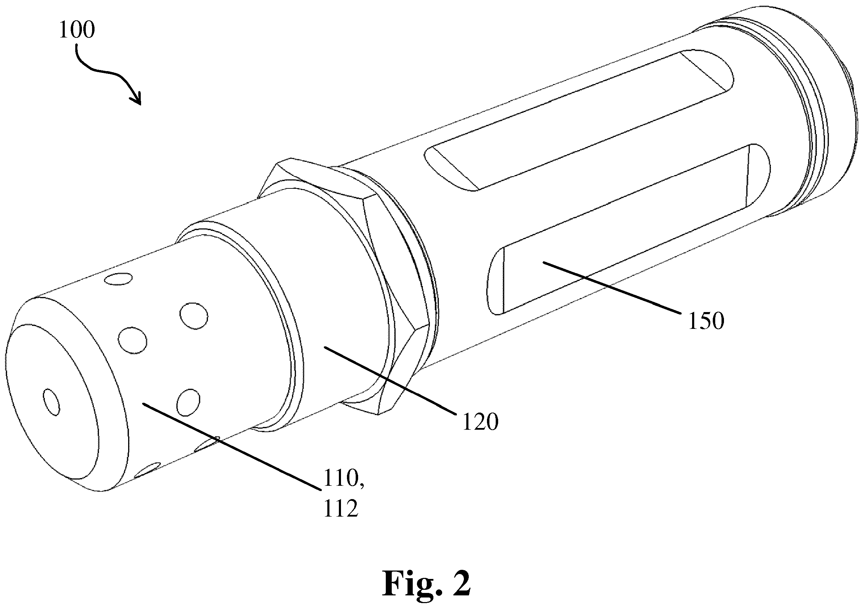

In der **Fig. 2** ist eine alternative Ausgestaltung der ersten Ausführungsform einer erfindungsgemäßen Vorrichtung 100 zur Bestimmung und/oder Überwachung des Zustandes eines Transformatorenöls gezeigt, welche ebenfalls in der Form eines Sticks 100 ausgebildet ist. Wie deutlich in der Fig. 2 zu erkennen ist, weist der Stick 100 einen Sensorbereich 110 mit einer Ultraschall-Sende- und/oder -Empfangseinrichtung oder einer Resonanzkammer 112 auf. Diese ist zu erkennen, da in der alternativen Ausgestaltung der ersten Ausführungsform keine Schutzabdeckung gezeigt ist. Der Stick 100 weist zudem einen Befestigungsbereich 120 zur sicheren Befestigung des Sticks 100 an einem Transformator auf, welcher in dieser Alternative beispielsweise als 1,0-Zoll-Rohrgewinde ausgebildet ist. Ebenfalls ist ein Gehäuse 150 umfasst.

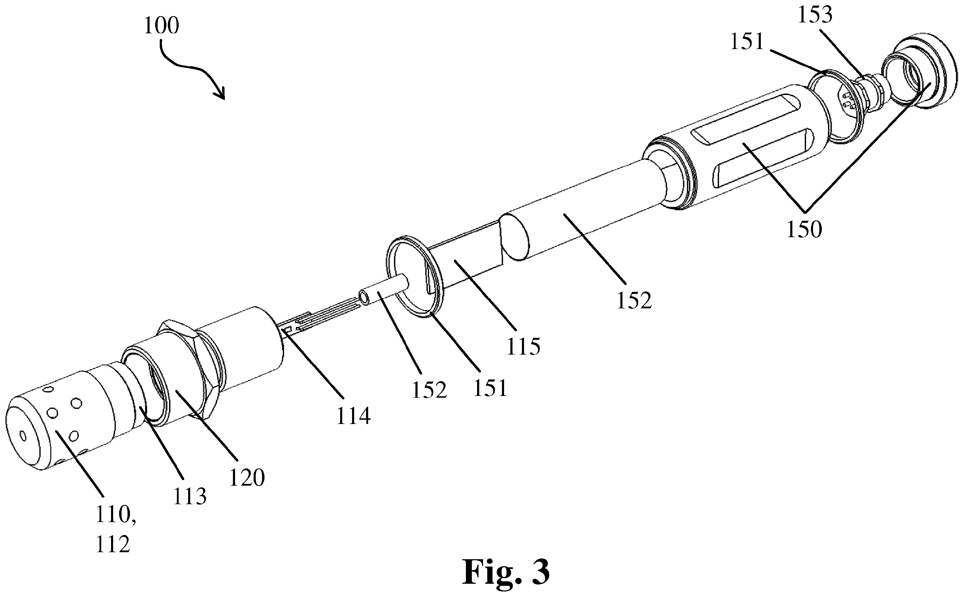

In der **Fig. 3** ist eine Explosionsdarstellung der alternativen Ausgestaltung der ersten Ausführungsform der erfindungsgemäßen Vorrichtung 100 nach Fig. 2 gezeigt, welche die innere Struktur der Vorrichtung 100 zeigt. Wie deutlich zu erkennen ist, sind kritische und empfindliche Komponenten der Vorrichtung 100 durch ein robustes und ausgeklügeltes Zusammenspiel von Anordnung und Gehäuseelementen 150, 151 und 152 abgedeckt und geschützt. Dieser Stick 100 weist ebenfalls einen Sensorbereich 110 auf sowie eine aufgrund der nicht dargestellten Schutzabdeckung sichtbare Resonanzkammer 112. Des Weiteren ist ein akustischer Wandler 113 und ein Befestigungsbereich 120 umfasst, welcher, wie ebenfalls in Fig. 2 gezeigt, beispielsweise in der Form eines 1,0-Zoll-Rohrgewindes ausgebildet ist.

Des Weiteren ist in Fig. 3 deutlich zu erkennen, dass ein Feuchte- und/oder Temperatur-Sensor 114 sowie der Resonator in der Resonanzkammer 112 und dem akustischen Wandler 113 aufgenommen sind. Zudem ist die entsprechende Elektronik 115 umfasst. Die Elektronik 115 ist von einer Isolierung 152, beispielsweise aus einem Kunststoff, umgeben. Das Gehäuse 150 umfasst zudem mehrere Isolierelemente 152, welche beispielsweise aus Kunststoff herstellbar sind. Des Weiteren sind mehrere Distanzringe 151 und eine Kabelbuchse 153 umfasst, um, beispielsweise über Modbus, eine Verbindung mit der Sensorelektronik des Sticks 100 zu ermöglichen.

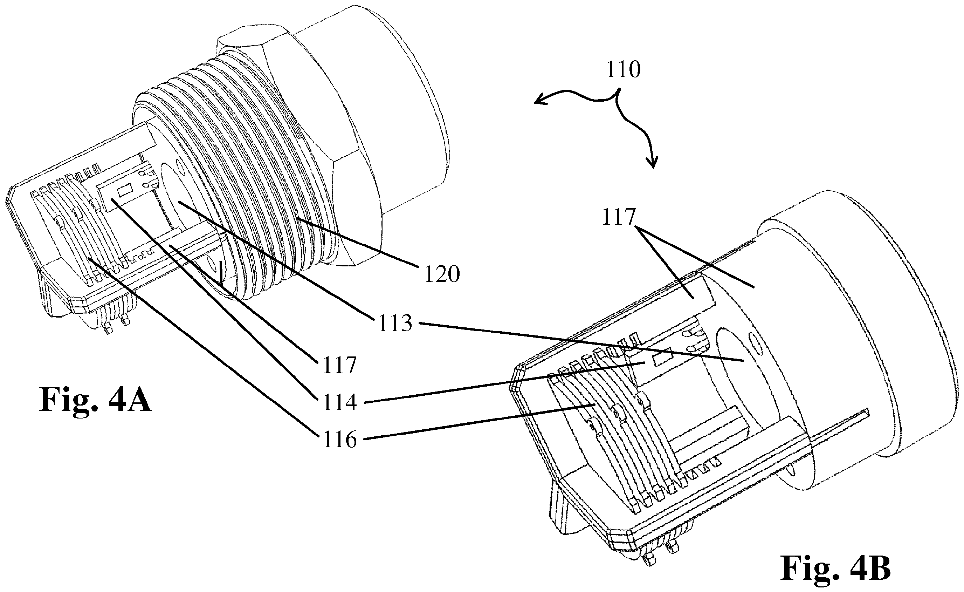

In der **Fig. 4** sind zwei alternative Ausgestaltungen einer ersten Ausführungsform des Sensorbereichs 110 der erfindungsgemäßen Vorrichtung 100 gezeigt. Wie deutlich in **Fig. 4a** und **Fig. 4b** zu erkennen ist, umfasst der Sensorbereich 110 mehrere Kondensatorplatten 116, welche Teil eines dielektrischen Sensors sind. Des Weiteren ist ein Feuchte- und/oder Temperatur-Sensor 114 und ein akustischer Wandler mit Resonanzkammer 113 umfasst. Das Halteelement 117 ist beispielsweise isolierend und aus einem Kunststoff herstellbar. In der Fig. 4a ist zudem ein Befestigungsbereich 120 dargestellt, welcher beispielsweise in der Form eines 1,5-Zoll-Rohrgewindes, ebenso wie in Fig. 1, ausgebildet ist.

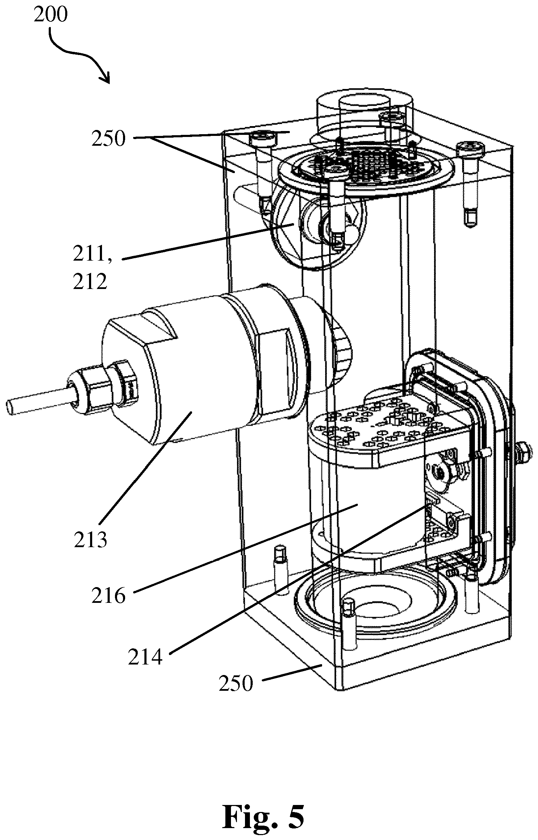

In der **Fig. 5** ist eine zweite Ausführungsform einer erfindungsgemäßen Vorrichtung 200 zur Bestimmung und/oder Überwachung des Zustandes eines Transformatorenöls gezeigt, welche in der Form einer Messkammer 200 ausgebildet ist. Wie deutlich in Fig. 5 zu erkennen ist, weist die Messkammer 200 mehrere Sensorbereiche auf, so ist beispielsweise in einem Sensorbereich ein Dichte- und/oder Viskositäts-Sensor 211 und/oder ein optischer Sensor 212 angeordnet. In einem weiteren Sensorbereich ist ein akustischer Sensor 213 angeordnet sowie weiterhin ein dielektrischer Sensor 216 und ein Feuchte- und/oder Temperatur-Sensor 214. Die Messkammer 200 weist zudem ein Gehäuse 250 auf.

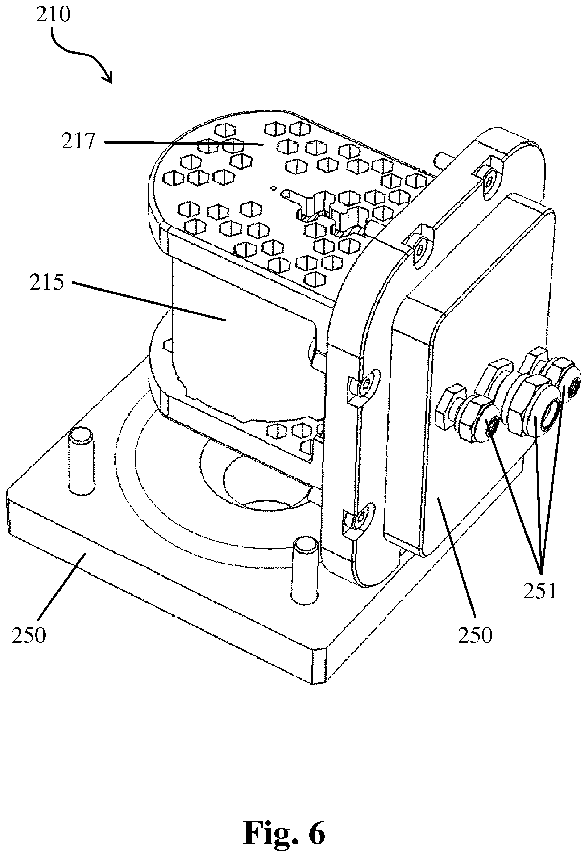

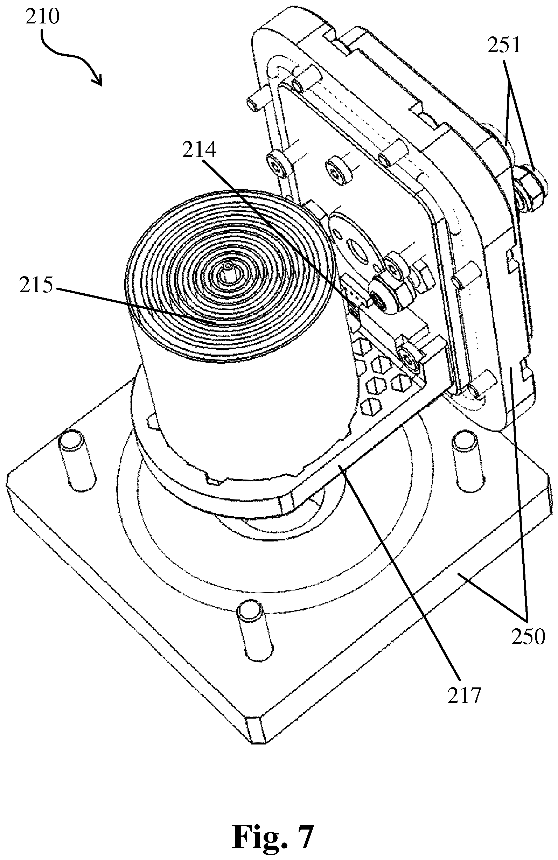

In den **Fig. 6** und **Fig. 7** ist einer der Sensorbereiche 210 der Messkammer nach Fig. 5 im Detail dargestellt. Wie deutlich in den Fig. 6 und 7 dargestellt, ist ein Halteelement 217 umfasst, welches isolierend ist und beispielsweise aus einem Kunststoff herstellbar ist. Zudem sind in beiden Figuren deutlich die Kondensatorplatten 215(ausgeführt als Zylinderkondensator aus konzentrischen Rohren) zu erkennen, welche Teil des dielektrischen Sensors sind. In der Fig. 7 ist zudem der Feuchte- und/oder Temperatur-Sensor 214 zu erkennen. Weiterhin ist in den Fig. 6 und Fig. 7 das der Isolierung der Messkammer aus Fig. 5 dienende Gehäuse 250 nebst den Kabeldurchführungen 251 zur Verbindung mit dieser sichtbar.

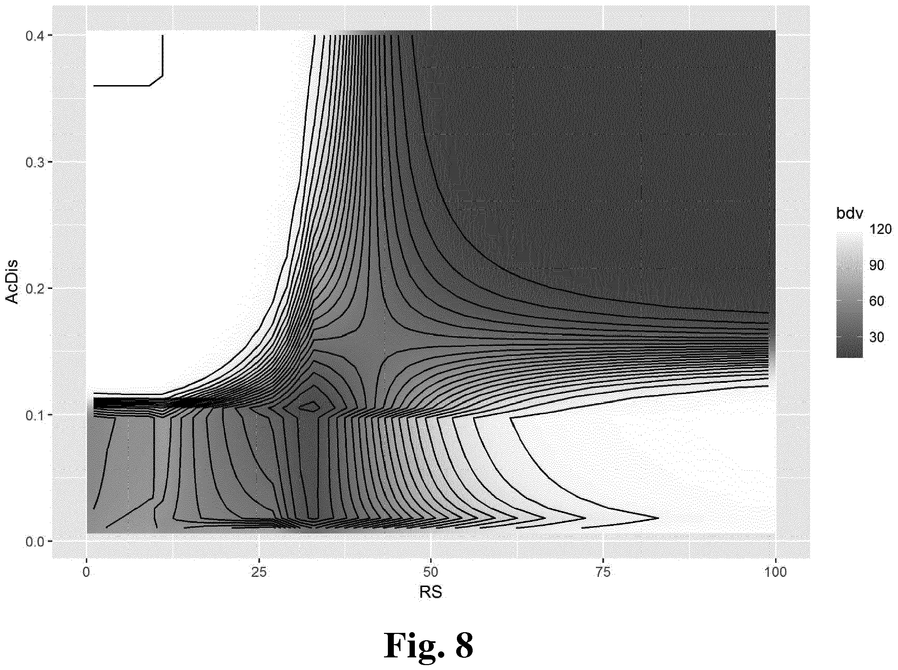

In der **Fig. 8** ist eine graphische Darstellung in Form einer Matrix-Grafik für ein beispielhaftes Berechnungsbeispiel der Bestimmung der Durchschlagspannung (BDV; eng. *break down voltage*) eines Transformatorenöls basierend auf einer 2-dimensionalen Funktion aus relativer Feuchte (RS; eng. *relative saturation*) und akustischem Ungleichgewicht (AcDis; eng. *acustic disbalance*) gezeigt. Die in der Matrix dargestellten Isogene entsprechen je 5 [kV].

In der ersten Stufe der 2-dimensionalen Funktion wird die Funktion *bdvL (RS, AcDis)* berechnet, welche von der Hauptfunktion *h(x)* abhängig ist. Diesbezüglich gilt die folgende Formel (1):

$$h(x) = \begin{cases} x & wenn\ x \geq 0 \\ 0 & wenn\ x < 0 \end{cases}$$

wobei

*h* die Hauptfunktion, und
*x* der Argumentenwert ist.

Gemäß Formel (1) hat die Hauptfunktion unterschiedliche Argumente. Wenn der Argumentenwert $x \geq 0$ ist, dann übernimmt die Funktion diesen Wert. Ist der Argumentenwert $x < 0$, dann ist der Wert null und dieses Gleichungsglied wird gelöscht.

Ausgehend von dieser Formel (1) ist nachfolgend in Formel (2) ein beispielhaftes Berechnungsbeispiel der Bestimmung der Durchschlagspannung (BDV) gezeigt. Die Formel (2) lautet:

$$
\begin{aligned}
\mathrm{bdvL\ (RS, AcDis)} = {}& -0.10 - \\
& 0.23\mathrm{h}(0.096885 - \log_{10}(\mathrm{RS})) - \\
& 96.79\ \mathrm{h}(\log_{10}(\mathrm{RS}) - 0.986885) - \\
& 9.38\ \mathrm{h}(\log_{10}(\mathrm{RS}) - 1.03756) - \\
& 19.27\ \mathrm{h}(\log_{10}(\mathrm{RS}) - 1.43403) + \\
& 30.27\ \mathrm{h}(\log_{10}(\mathrm{RS}) - 1.51121) + \\
& 0.21\ \mathrm{h}(-0.987312 - \log_{10}(\mathrm{AcDis})) + \\
& 67.11\ \mathrm{h}(\log_{10}(\mathrm{AcDis}) + 0.987312) - \\
& 169.59\ \mathrm{h}(\log_{10}(\mathrm{RS}) - 0.986885)*\ \mathrm{h}(\log_{10}(\mathrm{AcDis}) + 1.44532) + \\
& 169.36\ \mathrm{h}(\log_{10}(\mathrm{RS}) - 0.986885 * \mathrm{h}(-1.44532 - \log_{10}(\mathrm{AcDis})) - \\
& 119.70\ \mathrm{h}(\log_{10}(\mathrm{RS}) - 0.986885) * \mathrm{h}(\log_{10}(\mathrm{AcDis}) + 0.996463) + \\
& 179.58\ \mathrm{h}(\log_{10}(\mathrm{RS}) - 0.986885 * \mathrm{h}(\log_{10}(\mathrm{AcDis}) + 1.99022) + \\
& 0.02\ \mathrm{h}(1.04391 - \log_{10}(\mathrm{RS})) * \mathrm{h}(-0.987312 - \log_{10}(\mathrm{AcDis})) + \\
& 13.10\ \mathrm{h}(\log_{10}(\mathrm{RS}) - 1.04391) * \mathrm{h}(-0.987312 - \log_{10}(\mathrm{AcDis})) + \\
& 11.55\ \mathrm{h}(\log 10(\mathrm{RS}) - 1.43403) * \mathrm{h}(\log 10(\mathrm{AcDis}) + 2.00147
\end{aligned}
$$

wobei

*bdvL* ein nicht normierter Zwischenwert der Durchschlagspannung,
*h* die Hauptfunktion mit den Argumentenwerten x,

wobei
*x = RS* die relative Feuchte, und
*x = AcDis* die akustische Disbalance ist.

In der zweiten Stufe der 2-dimensionalen Funktion wird der mittels Formel (2) berechnete Wert *bdvL* gemäß der geltenden Norm DIN EN 60243-1:2012-05 normiert (siehe "Elektrische Durchschlagfestigkeit von isolierenden Werkstoffen - Prüfverfahren - Teil 1: Prüfungen bei technischen Frequenzen" (IEC 112/199/CDV:2011)). Die Normierung erfolgt gemäß der Formel (3):

$$BDV = 10 + \frac{110}{1 + \exp(-bdvL)} \; [kV]$$

wobei

*bdvL* ein nicht normierter Zwischenwert der Durchschlagspannung, und
*BDV* die Durchschlagspannung ist.

Weitere Informationen sind dem bekannten Standardwerk Friedman (1991) Multivariate Adaptive Regression Splines (with discussion) Annals of Statistics 19/1, 1-141, zu entnehmen (https://statistics.stanford.edu/research/multivariate-adaptive-regressionsplines).

**Bezugszeichenliste**

| | |
|---|---|
| 100 | Stick |
| 110 | Sensorbereich |
| 111 | Schutzabdeckung |
| 112 | Resonanzkammer |
| 113 | Akustischer Wandler |
| 114 | Feuchte- und/oder Temperatur-Sensor |
| 115 | Elektronik |
| 116 | Kondensatorplatten |
| 117 | Halteelement |
| 120 | Befestigungsbereich |
| 150 | Gehäuse |
| 151 | Distanzring |
| 152 | Isolierelement |
| 153 | Kabelbuchse |
| | |
| 200 | Messkammer |
| 210 | Sensorbereich |
| 211 | Dichte- und/oder Viskositäts-Sensor |
| 212 | Optischer Sensor |
| 213 | Akustischer Sensor |
| 214 | Feuchte- und/oder Temperatur-Sensor |
| 215 | Kondensatorplatten |
| 216 | Dielektrischer Sensor |
| 217 | Halteelement |
| 250 | Gehäuse |
| 251 | Kabeldurchführung |

**Patentansprüche**

1. Verfahren zur Bestimmung und/oder Überwachung des Zustandes eines Transformatorenöls, **gekennzeichnet durch** das

   a) Durchführen einer akustischen Spektroskopie des Transformatorenöls, wobei mehrere Ultraschall-Sendesignale unterschiedlicher Frequenz und/oder Amplitude in das Transformatorenöl ausgesendet und entsprechende reflektierte und/oder transmittierte Ultraschall-Empfangssignale unterschiedlicher Frequenz und/oder Amplitude nach Durchlaufen des Transformatorenöls empfangen werden; und

   b) Vergleichen der Ultraschall-Sendesignale mit den entsprechenden Ultraschall-Empfangssignalen, wobei eine für das Transformatorenöl charakteristische n-dimensionale Funktion ermittelt wird; und

   c) Zuordnen der ermittelten charakteristischen n-dimensionalen Funktion aus Schritt b) zu einer für Transformatorenöle bekannten Referenz-Funktion entsprechender Dimension, wobei ein Referenz-Transformatorenöl bestimmt wird; und

   d) Erfassen eines ersten Wertes mindestens einer charakteristischen physikalischen Eigenschaft des Transformatorenöls; und

   e) Vergleichen des ersten Wertes mit einem entsprechenden Wert des Referenz-Transformatorenöls; und

   f) Ermitteln des Zustandes des Transformatorenöls basierend auf dem in Schritt e) durchgeführten Vergleich.

2. Verfahren nach Anspruch 1,
   wobei das Verfahren zusätzlich umfasst:
   g) Darstellen der in Schritt f) durchgeführten Ermittlung.

3. Verfahren nach einem der vorhergehenden Ansprüche,
   wobei nach dem Schritt d) ein zusätzlicher Schritt d1) umfasst ist: d1) Erfassen mindestens eines zweiten Wertes mindestens einer charakteristischen physikalischen Eigenschaft des Transformatorenöls.

4. Verfahren nach einem der vorhergehenden Ansprüche,
   wobei mittels akustischer Spektroskopie die Dichte, die Viskosität, die relative und/oder die absolute Menge eines Inhibitors und/oder einer Säure des Transformatorenöls und/oder ein Gemisch daraus bestimmt werden.

5. Verfahren nach einem der vorhergehenden Ansprüche,
   wobei die charakteristische physikalische Eigenschaft die Schallgeschwindigkeit, die Dichte, die Farbe, der Brechungsindex, die Schallabsorption, die Temperatur, die Oberflächenspannung, die Viskosität, die relative und/oder absolute Feuchte, der Verlustfaktor, der Säuregehalt, die elektrische Konstante, die elektrische Leitfähigkeit und/oder die Konzentration mindestens eines Fluides ist.

6. Verfahren nach einem der vorhergehenden Ansprüche,
   wobei der Zustand ausgewählt ist aus der Liste der restlichen Nutzungsdauer, dem Alterungszustand, der Durchschlagspannung und/oder dem Bevorstehen eines Transformatorenöl-Wechsels oder einer Transformatorenöl-Regeneration.

7. Vorrichtung (100, 200) zur Bestimmung und/oder Überwachung des Zustandes eines Transformatorenöls nach einem der vorhergehenden Verfahrensansprüche, **gekennzeichnet durch**

   a) ein erstes Mittel (110, 112, 113, 210, 211, 212, 213) zum Durchführen einer akustischen Spektroskopie des Transformatorenöls, wobei das erste Mittel (110, 112, 113, 210, 211, 212, 213) eine Ultraschall-Sendeeinrichtung zum Aussenden mehrerer Ultraschall-Sendesignale unterschiedlicher Frequenz und/oder Amplitude in das Transformatorenöl und eine Ultraschall-Empfangseinrichtung zum Empfangen entsprechender reflektierter und/oder transmittierter Ultraschall-Empfangssignale unterschiedlicher Frequenz und/oder Amplitude nach Durchlaufen des Transformatorenöls umfasst; und

   b) eine erste Evaluierungseinheit zum Vergleichen der Ultraschall-Sendesignale mit den entsprechenden Ultraschall-Empfangssignalen, wobei eine für das Transformatorenöl charakteristische n-dimensionale Funktion ermittelbar ist; und

   c) eine erste Auswerteeinheit zum Zuordnen der ermittelten charakteristischen n-dimensionalen Funktion aus Schritt b) zu einer für Transformatorenöle bekannten Referenz-Funktion entsprechender Dimension, wobei ein Referenz-Transformatorenöl bestimmbar ist; und

   d) ein zweites Mittel (110, 114, 116, 210, 214, 215, 216) zum Erfassen eines ersten Wertes mindestens einer charakteristischen physikalischen Eigenschaft des Transformatorenöls; und

e) eine zweite Evaluierungseinheit zum Vergleichen des ersten Wertes mit einem entsprechenden Wert des Referenz-Transformatorenöls; und

f) eine zweite Auswerteeinheit zum Ermitteln des Zustandes des Transformatorenöls basierend auf dem in Schritt e) durchgeführten Vergleich.

8. Vorrichtung (100, 200) nach Anspruch 7,
wobei zusätzlich eine Ausgabeeinheit zur Darstellung der mittels der zweiten Ausgabeeinheit durchgeführten Ermittlung umfasst ist.

9. Vorrichtung (100, 200) nach einem der Ansprüche 7 oder 8,
wobei ein zusätzliches Mittel (110, 112, 113, 114, 116, 210, 211, 212, 213, 214, 215, 216) zum Erfassen mindestens eines zweiten Wertes mindestens einer charakteristischen physikalischen Eigenschaft des Transformatorenöls umfasst ist.

10. Vorrichtung (100, 200) nach einem der Ansprüche 7 bis 9,
wobei mittels des ersten Mittels (110, 112, 113, 210, 211, 212, 213) die Dichte, die Viskosität, die relative und/oder die absolute Menge eines Inhibitors und/oder einer Säure des Transformatorenöls und/oder ein Gemisch daraus bestimmbar sind.

11. Vorrichtung (100, 200) nach einem der Ansprüche 7 bis 10,
wobei das erste Mittel (110, 112, 113, 210, 211, 212, 213) und/oder das zweite Mittel (110, 114, 116, 210, 214, 215, 216) die Schallgeschwindigkeit, die Dichte, die Farbe, der Brechungsindex, die Schallabsorption, die Temperatur, die Oberflächenspannung, die Viskosität, die relative und/oder absolute Feuchte, den Verlustfaktor, den Säuregehalt, die elektrische Konstante, die elektrische Leitfähigkeit und/oder die Konzentration mindestens eines Fluides erfasst.

12. Vorrichtung (100, 200) nach einem der Ansprüche 7 bis 11,
wobei der Zustand ausgewählt ist aus der Liste aus der restlichen Nutzungsdauer, dem Alterungszustand, der Durchschlagspannung und/oder dem Bevorstehen eines Transformatorenöl-Wechsels oder einer Transformatorenöl-Regeneration.

13. Vorrichtung (100, 200) nach einem der Ansprüche 7 bis 12,
wobei das erste Mittel (110, 112, 113, 210, 211, 212, 213), das zweite Mittel (110, 114, 116, 210, 214, 215, 216), die erste Evaluierungseinheit, die zweite Evaluierungseinheit, die erste Auswerteeinheit, die zweite Auswerteeinheit und/oder die Ausgabeeinheit in einem Bauteil anordenbar sind.

14. Vorrichtung (100, 200) nach Anspruch 13, wobei das Bauteil eine Messkammer (200), ein Stick (100) und/oder ein Adapter ist.

15. Vorrichtung (100, 200) nach einem der Ansprüche 7 bis 14, wobei die Vorrichtung (100, 200) eine Heizeinrichtung umfasst.

16. Vorrichtung in der Hochspannungstechnik,
insbesondere ein Transformator, ein Kondensator, eine Petersenspule und/oder ein Schalter, umfassend Transformatorenöl, eine Vorrichtung (100,200) nach einem der Ansprüche 7-15 und eine Einrichtung zur Verbindung mit der Vorrichtung (100, 200) nach einem der Ansprüche 7 bis 15, wobei die Verbindung direkt erfolgt.

**Claims**

1. A method for determining and/or monitoring the state of a transformer oil, **characterised by** the steps of

a) performing an acoustic spectroscopy of the transformer oil, multiple ultrasonic emission signals of different frequencies and/or amplitudes being emitted into the transformer oil and corresponding reflected and/or transmitted ultrasonic reception signals of different frequencies and/or amplitudes being received after having passed through the transformer oil; and

b) comparing the ultrasonic emission signals with the corresponding ultrasonic reception signals, an n-dimensional function characteristic of the transformer oil being ascertained; and

c) matching the ascertained characteristic n-dimensional function from step b) with a reference function of corresponding dimension known for transformer oils, a reference transformer oil being determined; and
d) registering a first value of at least one characteristic physical property of the transformer oil; and
e) comparing the first value with a corresponding value of the reference transformer oil; and
f) ascertaining the state of the transformer oil based on the comparison performed in step e).

2. The method according to claim 1,
wherein the method additionally comprises the step of: g) displaying the ascertainment performed in step f).

3. The method according to any one of the preceding claims,
wherein the method comprises an additional step d1) after step d):
d1) registering at least one second value of at least one characteristic physical property of the transformer oil.

4. The method according to any one of the preceding claims,
wherein the density, the viscosity, the relative and/or absolute amount of an inhibitor and/or of an acid of the transformer oil and/or a mixture thereof are determined by means of acoustic spectroscopy.

5. The method according to any one of the preceding claims,
wherein the characteristic physical property is the speed of sound, the density, the colour, the refractive index, the sound absorption, the temperature, the interfacial tension, the viscosity, the relative and/or absolute saturation, the loss factor, the acid number, the electric constant, the electrical conductivity and/or the concentration of at least one fluid.

6. The method according to any one of the preceding claims,
wherein the state is selected from the list of remaining useful life, state of health, breakdown voltage and/or imminence of a transformer oil change or of a transformer oil regeneration.

7. A device (100, 200) for determining and/or monitoring the state of a transformer oil according to any one of the preceding method claims, **characterised by**

a) a first medium (110, 112, 113, 210, 211, 212, 213) for performing an acoustic spectroscopy of the transformer oil, the first medium (110, 112, 113, 210, 211, 212, 213) comprising an ultrasonic emitter for emitting multiple ultrasonic emission signals of different frequencies and/or amplitudes into the transformer oil and an ultrasonic receiver for receiving corresponding reflected and/or transmitted ultrasonic reception signals of different frequencies and/or amplitudes after said ultrasonic reception signals have passed through the transformer oil; and
b) a first evaluating unit for comparing the ultrasonic emission signals with the corresponding ultrasonic reception signals, an n-dimensional function characteristic of the transformer oil being ascertained; and
c) a first analysing unit for matching the ascertained characteristic n-dimensional function from step b) with a reference function of corresponding dimension known for transformer oils, a reference transformer oil being determined; and
d) a second medium (110, 114, 116, 210, 214, 215, 216) for registering a first value of at least one characteristic physical property of the transformer oil; and
e) a second evaluating unit for comparing the first value with a corresponding value of the reference transformer oil; and
f) a second analysing unit for ascertaining the state of the transformer oil based on the comparison performed in step e).

8. The device (100, 200) according to claim 7,
wherein the device (100, 200) additionally comprises an output unit for displaying the ascertainment performed by means of the second output unit.

9. The device (100, 200) according to any one of claims 7 or 8,
wherein the device (100, 200) comprises an additional medium (110, 112, 113, 114, 116, 210, 211, 212, 213, 214, 215, 216) for registering at least one second value of at least one characteristic physical property of the transformer oil.

10. The device (100, 200) according to any one of claims 7 to 9,
wherein the density, the viscosity, the relative and/or absolute amount of an inhibitor and/or of an acid of the transformer oil and/or a mixture thereof is determined by means of the first medium (110, 112, 113, 210, 211, 212, 213).

**11.** The device (100, 200) according to any one of claims 7 to 10,
wherein the first medium (110, 112, 113, 210, 211, 212, 213) and/or the second medium (110, 114, 116, 210, 214, 215, 216) register the speed of sound, the density, the colour, the refractive index, the sound absorption, the temperature, the interfacial tension, the viscosity, the relative and/or absolute saturation, the loss factor, the acid number, the electric constant, the electrical conductivity and/or the concentration of at least one fluid.

**12.** The device (100, 200) according to any one of the claims 7 to 11,
wherein the state is selected from the list of remaining useful life, state of health, breakdown voltage and/or imminence of a transformer oil change or of a transformer oil regeneration.

**13.** The device (100, 200) according to any one of claims 7 to 12,
wherein the first medium (110, 112, 113, 210, 211, 212, 213), the second medium (110, 114, 116, 210, 214, 215, 216), the first evaluating unit, the second evaluating unit, the first analysing unit, the second analysing unit and/or the output unit are disposed in one component.

**14.** The device (100, 200) according to claim 13, wherein the component is a measuring chamber (200), a stick (100) and/or an adapter.

**15.** The device (100, 200) according to any one of claims 7 to 14,
wherein the device (100, 200) comprises a heating device.

**16.** A device of high-voltage technology,
in particular a transformer, a capacitor, a Petersen coil and/or a switch, comprising transformer oil, a device (100, 200) according to any one of claims 7 to 15 and a means for connecting the device to the device (100, 200) according to any one of claims 7 to 15, the connection being a direct connection.

## Revendications

**1.** Procédé pour déterminer et/ou contrôler l'état d'une huile de transformateur, **caractérisé par** les étapes suivantes :

a) effectuer une spectroscopie acoustique de l'huile de transformateur, plusieurs signaux d'émission ultrasonores d'une fréquence et/ou amplitude différentes étant émis dans l'huile de transformateur et des signaux de réception ultrasonores correspondants réfléchis et/ou transmis d'une fréquence et/ou amplitude différentes étant reçus après avoir traversé l'huile de transformateur ; et
b) comparer les signaux d'émission ultrasonores avec les signaux de réception ultrasonores correspondants, une fonction à n dimensions caractéristique pour l'huile de transformateur étant déterminée ; et
c) attribuer la fonction à n dimensions caractéristique déterminée de l'étape b) à une fonction de référence connue pour des huiles de transformateur d'une dimension correspondante, une huile de transformateur de référence étant déterminée ; et
d) saisir une première valeur d'au moins une propriété physique caractéristique de l'huile de transformateur ; et
e) comparer la première valeur avec une valeur correspondante de l'huile de transformateur de référence ; et
f) déterminer l'état de l'huile de transformateur sur la base de la comparaison effectuée dans l'étape e).

**2.** Procédé selon la revendication 1,
le procédé comprenant additionnellement l'étape suivante : d) afficher la détermination effectuée dans l'étape f).

**3.** Procédé selon l'une quelconque des revendications précédentes,
dans lequel le procédé comprend une étape supplémentaire d1) après l'étape d) :
d1) saisir au moins une deuxième valeur d'au moins une propriété physique caractéristique de l'huile de transformateur.

**4.** Procédé selon l'une quelconque des revendications précédentes, dans lequel la densité, la viscosité, la quantité relative et/ou absolue d'un inhibiteur et/ou d'un acide de l'huile de transformateur et/ou un mélange de ceux-ci sont déterminés à l'aide d'une spectroscopie acoustique.

**5.** Procédé selon l'une quelconque des revendications précédentes, dans lequel la propriété physique caractéristique est la vitesse du son, la densité, la couleur, l'indice de réfraction, l'absorption acoustique, la température, la tension superficielle, la viscosité, la saturation relative et/ou absolue, le facteur de perte, la teneur en acide, la constante électrique, la conductivité électrique et/ou la concentration d'au moins un fluide.

6. Procédé selon l'une quelconque des revendications précédentes, dans lequel l'état est choisi de la liste comprenant la vie utile restante, l'état de santé, la tension de claquage et/ou l'imminence d'un changement de l'huile de transformateur ou d'une régénération de l'huile de transformateur.

7. Dispositif (100, 200) pour déterminer et/ou contrôler l'état d'une huile de transformateur selon l'une quelconque des revendications de procédé précédentes, **caractérisé par**

   a) un premier moyen (110, 112, 113, 210, 211, 212, 213) pour effectuer une spectroscopie acoustique de l'huile de transformateur, le premier moyen (110, 112, 113, 210, 211, 212, 213) comprenant un moyen d'émission d'ultrasons pour émettre plusieurs signaux d'émission ultrasonores d'une fréquence et/ou amplitude différentes dans l'huile de transformateur et un moyen de réception d'ultrasons pour recevoir des signaux de réception ultrasonores correspondants réfléchis et/ou transmis d'une fréquence et/ou amplitude différentes après que lesdits signaux de réception ultrasonores ont traversé l'huile de transformateur ; et
   b) une première unité d'analyse pour comparer les signaux d'émission ultrasonores avec les signaux de réception ultrasonores correspondants, une fonction à n dimensions caractéristique pour l'huile de transformateur étant déterminable ; et
   c) une première unité d'évaluation pour attribuer la fonction à n dimensions caractéristique déterminée dans l'étape b) à une fonction de référence connue pour des huiles de transformateur d'une dimension correspondante, une huile de transformateur de référence étant déterminable ; et
   d) un deuxième moyen (110, 114, 116, 210, 214, 215, 216) pour saisir une première valeur d'au moins une propriété physique caractéristique de l'huile de transformateur ; et
   e) une deuxième unité d'analyse pour comparer la première valeur avec une valeur correspondante de l'huile de transformateur de référence ; et
   f)) une deuxième unité d'évaluation pour déterminer l'état de l'huile de transformateur sur la base de la comparaison effectuée dans l'étape e).

8. Dispositif (100, 200) selon la revendication 7,
   dans lequel le dispositif (100, 200) comprend additionnellement une unité de sortie pour afficher la détermination effectuée à l'aide de la deuxième unité de sortie.

9. Dispositif (100, 200) selon la revendication 7 ou 8,
   dans lequel le dispositif (100, 200) comprend un moyen additionnel (110, 112, 113, 114, 116, 210, 211, 212, 213, 214, 215, 216) pour saisir au moins une deuxième valeur d'au moins une propriété physique caractéristique de l'huile de transformateur.

10. Dispositif (100, 200) selon l'une quelconque des revendications 7 à 9,
    dans lequel la densité, la viscosité, la quantité relative et/ou absolue d'un inhibiteur et/ou d'un acide de l'huile de transformateur et/ou un mélange de ceux-ci sont déterminables à l'aide du premier moyen (110, 112, 113, 210, 211, 212, 213).

11. Dispositif (100, 200) selon l'une quelconque des revendications 7 à 10,
    dans lequel le premier moyen (110, 112, 113, 210, 211, 212, 213) et/ou le deuxième moyen (110, 114, 116, 210, 214, 215, 216) saisissent la vitesse du son, la densité, la couleur, l'indice de réfraction, l'absorption acoustique, la température, la tension superficielle, la viscosité, la saturation relative et/ou absolue, le facteur de perte, la teneur en acide, la constante électrique, la conductivité électrique et/ou la concentration d'au moins un fluide.

12. Dispositif (100, 200) selon l'une quelconque des revendications 7 à 11,
    dans lequel l'état est choisi de la liste comprenant la vie utile restante, l'état de santé, la tension de claquage et/ou l'imminence d'un changement de l'huile de transformateur ou d'une régénération de l'huile de transformateur.

13. Dispositif (100, 200) selon l'une quelconque des revendications 7 à 12,
    dans lequel le premier moyen (110, 112, 113, 210, 211, 212, 213), le deuxième moyen (110, 114, 116, 210, 214, 215, 216), la première unité d'analyse, la deuxième unité d'analyse, la première unité d'évaluation, la deuxième unité d'évaluation et/ou l'unité de sortie sont capables d'être disposés dans un composant.

**14.** Dispositif (100, 200) selon la revendication 13, dans lequel le composant est une chambre de mesure (200), un bâton (100) et/ou un adaptateur.

**15.** Dispositif (100, 200) selon l'une quelconque des revendications 7 à 14,
dans lequel le dispositif (100, 200) comprend un dispositif de chauffage.

**16.** Dispositif de la technique de la haute tension,
notamment un transformateur, un condensateur, une bobine Petersen et/ou un commutateur, comprenant de l'huile de transformateur, un dispositif (100, 200) selon l'une quelconque des revendications 7 à 15 et un moyen pour la connexion du dispositif au dispositif (100, 200) selon l'une quelconque des revendications 7 à 15, la connexion étant une connexion directe.

**Fig. 1**

EP 3 513 155 B1

EP 3 513 155 B1

**Fig. 2**

**Fig. 3**

110

117

120

113

117

114

116

**Fig. 4A**

117

**Fig. 4B**

**Fig. 5**

**Fig. 6**

**Fig. 7**

Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102015122926
- DE 19706486